# FASCICULE DE BREVET EUROPEEN

(11) **EP 2 740 444 B1**
(45) Date de publication et mention de la délivrance du brevet: **08.05.2019**
(21) Numéro de dépôt: 13196189.8
(22) Date de dépôt: 09.12.2013
(51) Int. Cl.: A61F 2/30, A61F 2/34

(54) **Implant articulaire de hanche a structure ternaire**
Hüftgelenkimplantat mit ternärer Struktur
Hip joint implant with ternary structure

(30) Priorité: 10.12.2012 FR 1261822
(43) Date de publication de la demande: 11.06.2014
(73) Titulaire: Dedienne Sante, 34130 Mauguio (FR)
(72) Inventeur: Setti, Yves, 30230 Bouillargues (FR)
(74) Mandataire: Jeannet, Olivier

(56) Documents cités:
- EP-A1- 1 421 918
- WO-A1-02/087476
- WO-A1-2011/058519
- WO-A2-01/24739
- DE-A1- 19 904 436
- US-A- 4 888 024
- US-A- 6 087 553

## Description

### Domaine technique :

La présente invention appartient au domaine des dispositifs prothétiques et plus particulièrement des prothèses articulaires de hanche.

Elle a pour objet un implant articulaire comprenant une cupule et un insert, entre lesquels est interposé un élément intermédiaire en matériau polymère, ces trois éléments étant indissociablement liés par des interfaces interpénétrées.

### Etat de la technique :

La plupart des prothèses osseuses implantées sur des patients concernent les jonctions articulaires. Celles-ci sont particulièrement sollicitées au cours des activités humaines de toutes natures, alors qu'elles comptent parmi les structures les plus complexes du corps humain. Les prothèses articulaires qui s'y substituent, comprennent de par leur fonction, plusieurs éléments prothétiques associés et coopérant entre eux pour assurer la mobilité du membre. Toutes les articulations peuvent être concernées, et en premier lieu la hanche, le fémur étant mobile dans le bassin, mais aussi le genou où le tibia vient s'appuyer sur le fémur, avec souvent une pièce intermédiaire. Dans le cas des articulations des membres inférieurs, les éléments en contact doivent en outre être à même de supporter le poids du corps, ce qui ajoute aux contraintes mécaniques à satisfaire.

Dans la plupart des prothèses articulaires, on trouve un implant qui fait fonction d'élément d'appui destiné à coopérer avec un élément mobile de la prothèse. Dans la présente demande, ces éléments (d'appui et mobile) sont ainsi désignés par soucis de clarté, du fait de la fonction principale qu'ils remplissent dans la présente invention. L'un est essentiellement mobile, cette mobilité s'entendant de manière relative par rapport à l'autre élément, qui par opposition est supposé fixe et va avoir essentiellement une fonction d'appui. On note qu'en orthopédie, l'élément mobile n'a pas d'accroche osseuse. Par exemple, on considère que le cotyle de la hanche sert essentiellement d'appui à la partie proximale mobile du fémur lorsque celui-ci se déplace. Dans le cas du genou, l'élément mobile est le condyle fémoral, qui se déplace en frottant sur un élément en polyéthylène fixé au tibia.

La mise en place d'une prothèse totale de hanche est une intervention chirurgicale réalisée de façon assez courante. La hanche est l'une des articulations les plus sollicitées. Elle relie l'os du bassin (os iliaque) au fémur. Le bassin comporte une cavité articulaire, appelée cotyle, qui reçoit la tête du fémur. Lors d'une arthroplastie totale de la hanche, le cotyle doit être débarrassé de tout cartilage et os arthritiques (on dit que le cotyle est resurfacé), pour recevoir un cotyle prothétique (c'est ici l'élément d'appui), qui adopte la forme générale d'une coque et qui est apte à accueillir la tête sphérique de l'implant fémoral complémentaire (qui est l'élément mobile). Comme tout corps étranger introduit dans l'organisme, tout doit être mis en oeuvre pour que la prothèse soit tolérée au maximum par l'organisme. En particulier, l'usure et la dégradation des matériaux risquant de libérer des particules plus ou moins volumineuse dans l'organisme sont à minimiser et si possible à empêcher. Cette tolérance passe aussi bien par le choix des matériaux utilisés (titane, acier chirurgical céramique, matériaux de synthèse) que par la structure même de la prothèse. De plus, les implants doivent avoir une durée de vie la plus longue possible afin d'éviter au patient le remplacement de l'implant avec les opérations et les contraintes que cela implique. Le cotyle doit donc être solidement maintenu dans l'os iliaque par sa face proximale tandis que par sa face distale il coopère en mobilité avec la tête d'un fémur. Les composants les plus fragiles doivent être préservés au maximum de l'abrasion et des phénomènes d'usure.

Il existe plusieurs types de cotyles prothétiques utilisés pour l'arthroplastie de la hanche. Les premières prothèses, en céramique massique, furent posées sans fixation particulière, par simple encastrement. Puis des fixations par ciment furent utilisées. On connaît par exemple par US 4,888,024 une prothèse de hanche dans laquelle la cupule est recouverte d'une membrane flexible et d'une gaine en tissus entre lesquelles on injecte un ciment de fixation.

Vers le milieu des années 1970, est apparu le cotyle prothétique sans ciment, composé de deux éléments : une coquille prothétique métallique (la cupule) à l'intérieur de laquelle est placé un insert en polyéthylène ou en céramique. L'insert peut être réalisé en différents matériaux : polyéthylène, métal, céramique, selon une structure uniforme, ou une structure sandwich alternant polyéthylène et métal ou polyéthylène et céramique.

La cupule peut être vissée ou encastrée dans le bassin, tandis que l'insert est clipé dans la cupule. Dans ces systèmes l'association d'une cupule métallique, communément en titane, avec un insert en polyéthylène présente de nombreux avantages. La pose de la cupule est d'une grande facilité et sa stabilité est assurée par la possibilité d'une fixation renforcée par vissage dans l'os iliaque. L'insert est indépendant de la cupule, de sorte qu'en cas de besoin, il peut en être désolidarisé : une reprise de l'insert seul est possible. C'est ce type de cotyle qui est le plus utilisé à l'heure actuelle. Il présente cependant un certain nombre d'inconvénients, liés aux contraintes mécaniques importantes auxquelles la prothèse, qui supporte l'essentiel du poids du corps, est soumise. En effet, des mouvements microscopiques se produisent à l'interface cupule - insert, provoquant une usure du polyéthylène. Des débris de polyéthylène se détachent et finissent par provoquer une réaction inflammatoire autour de la prothèse, avec des conséquences potentiellement très graves pour le patient (lyse osseuse notamment). Pour éviter ce phénomène, des cotyles entièrement en polyéthylène, dits "Full PE", ont été proposés. Cependant, leur usure est relativement rapide et les praticiens sont réticents à employer des prothèses en polyéthylène, car leur durée de vie est trop courte pour une implantation sur des sujets de moins de 50 ans.

Une technique alternative a été utilisée, mettant en oeuvre deux pièces en céramique. Si on gagne nettement du point de vue de la réduction des débris, les caractéristiques élastiques sont insatisfaisantes du fait de la dureté élevée des deux pièces.

Plus récemment, pour remédier aux inconvénients de ces dispositifs à deux composants, des techniques alternatives ont été proposées qui consistent à utiliser des structures monoblocs dans lesquelles les éléments du cotyle sont réalisés dans des matériaux différents qui sont indissociablement liés entre eux.

Ce principe a été mis en oeuvre pour réaliser des structures dites "sandwich", dans lesquelles une coque externe réalisée en métal poreux est unie à une couche de polyéthylène pour former une cupule, laquelle reçoit un insert en céramique. Avec une telle association, on arrive à réduire la rigidité du cotyle par rapport à une association céramique-céramique et à empêcher les chocs indésirables entre la bordure de la coque en céramique et le col du fémur. Une structure sandwich intégrant un élément poreux a été réalisée qui a permis de fabriquer un cotyle comprenant une coque poreuse en tantale et du polyéthylène compressé, avec un insert en céramique. Cependant, des études ont montré que ce type de cotyle présentait un taux de fracture de l'insert très élevé. Il semble que doivent être incriminés un niveau de friction et un couple élevés, ce qui provoque une dislocation de l'insert et sa rupture (J. Bone Joint Surg. Am. 2007; 89:367-375).

Selon un principe voisin, le brevet US 6,087,553 décrit un implant articulaire réalisé dans un matériau à plusieurs composants : une couche de polyéthylène est moulée par compression dans une coque réalisée en matériau poreux. Le matériau poreux est une mousse de carbone, rigidifiée par un revêtement de tantale. Lors de la compression le polyéthylène pénètre dans la porosité de la coque sur une épaisseur de 1 à 3 mm, créant une interface extrêmement solide et immuable. La porosité très importante de la mousse de carbone durcie au tantale favorise la colonisation par l'os de la face proximale de la cupule. Dans cet implant, la coque poreuse doit être épaisse pour que le polyéthylène d'un côté et l'os de l'autre, puissent occuper deux plages distinctes de la porosité, ce qui représente plusieurs millimètres d'épaisseur. Les cotyles réalisés avec un matériau de ce type sont donc relativement massifs. Ceci contraint, soit à augmenter la taille du cotyle implanté, soit à réduire la taille de la tête du fémur, au risque d'accroître le nombre de luxations. La même critique peut être faite à l'égard de implant monobloc décrit dans EP 1421918 qui comporte trois couches en matériaux différents, avec une couche intermédiaire en polymère assurant la liaison entre une couche interne (insert) qui comporte des rainures de maintien et une couche externe (cupule) en métal poreux imprégné du polymère.

Or, il est indispensable que le diamètre externe du cotyle soit le plus petit possible afin de supprimer le moins d'os possible lors de sa pose, mais, en revanche, l'insert doit pouvoir accueillir une tête fémorale aussi volumineuse que possible pour éviter la luxation. On souhaite donc obtenir un cotyle de faible épaisseur.

En effet, pour le confort des patients, les prothèses doivent reproduire le plus fidèlement possible les conditions de mobilité anatomiques de l'articulation, en remplaçant au mieux les éléments osseux endommagés. En premier lieu, les conditions de mobilité doivent être obtenues en optimisant l'importance et la constance des surfaces d'appui des éléments en mouvement l'un par rapport à l'autre (l'élément fémoral sur l'élément intermédiaire pour le genou, le condyle sur la tête du fémur pour la hanche).

Mais la mobilité doit aussi être obtenue en préservant au mieux le capital osseux et en limitant les résections osseuses nécessaires à l'implantation de la prothèse. La conformation des prothèses doit donc concilier les contraintes dimensionnelles et celles liées à la mobilité et à la stabilité du dispositif.

De plus pour des raisons sanitaires, il est nécessaire d'éviter l'érosion du matériau polymère du dispositif (le plus souvent en polyéthylène) et la dispersion de débris et de particules dans l'organisme. Ce faisant, les autres exigences mentionnées précédemment doivent être respectées : bon amortissement des chocs, la fixation avec l'os favorisée.

Jusqu'à présent, ces objectifs n'ont pas pu être conciliés. Ainsi on constate que si les développements récents des travaux de recherche ont apporté certaines améliorations dans les systèmes monoblocs associant un implant métallique et un insert polymère, de nombreux inconvénients restent à surmonter pour fournir des prothèses solides, confortables et durables, quelle que soit l'articulation concernée. Ce sont les inconvénients précités que la présente invention vise à surmonter, tout en répondant aux contraintes inhérentes aux éléments prothétiques des surfaces articulaires.

### Exposé de l'invention :

Ainsi, un premier objet de l'invention est un implant articulaire destiné à être fixé dans une structure osseuse et à accueillir une tête prothétique mobile par rapport à ladite structure osseuse, comprenant :
i) une cupule en métal assurant la fixation de l'implant dans ladite structure osseuse,
ii) un insert assurant la réception de ladite tête prothétique, et
iii) un élément intermédiaire en matériau polymère, interposé entre la cupule et l'insert, l'implant étant caractérisé en ce que
   - l'insert présente à sa surface de contact avec l'élément intermédiaire un relief formant des cavités ou des interstices, la surface dudit élément intermédiaire épousant au moins en partie ledit relief, et
   - la cupule comprend,
j) deux couches superficielles présentant un relief formant des cavités ou des interstices, dont l'une, distale, présente une surface de contact avec l'élément intermédiaire, la surface dudit élément intermédiaire épousant au moins en partie ledit relief, et l'autre, proximale, est destinée à assurer la fixation de l'implant dans la structure osseuse, et
jj) une couche médiane dense qui constitue une cloison étanche entre lesdites deux couches superficielles,
de manière à réaliser des interfaces interpénétrées entre l'élément intermédiaire et la cupule d'une part, et entre l'élément intermédiaire et l'insert d'autre part.

La solution proposée repose sur la conception d'un dispositif permettant d'intégrer une épaisseur plus importante d'un élément intermédiaire pris pour ses propriétés de souplesse grâce à son module d'élasticité adapté tout en le solidarisant au sein du dispositif afin d'éviter toute friction et usure prématurée et tout risque sanitaire lié à l'érosion de celle-ci. Cet élément est lié avec les autres parties (insert et cupule) formant la prothèse de façon à former un ensemble indissociable.

Les termes de cupule et d'insert comme celui de cotyle évoqués plus loin, s'appliquent communément aux prothèses de hanches. La présente invention, qui sera décrite pour plus de simplicité dans ce contexte peut cependant s'appliquer à toute articulation du même type.

On désigne par cavité des structures de petite taille telle que les pores d'un matériau (la taille des pores pouvant être de l'ordre de quelques dizaines de nanomètres à quelques dizaines de micromètres par exemple). On réserve ici le terme d'interstice plutôt à des structures macroscopiques, de taille millimétrique, telles que rainures, nervures ou creux.

L'élément intermédiaire en polymère est en contact d'une part avec la cupule et d'autre part avec l'insert. Ce contact est réalisé par le fait que le polymère remplit par pénétration tout ou partie des cavités ou interstices. Il y a donc une interpénétration des matériaux au niveau de deux zones appelées "interfaces" qui assurent une fixation sans faille. Cette structure particulière de l'implant permet de supprimer tout micro déplacement entre les différents éléments. Sans frottement des pièces mobiles avec l'élément intermédiaire, le risque de produire de débris de matière dans l'organisme est ainsi totalement annulé.

Chacun des trois éléments i), ii) et iii) constitutifs de l'implant peut être réalisé de différentes manières et en différents matériaux. La partie de l'insert destinée à coopérer avec la tête fémorale prothétique est avantageusement réalisée dans un matériau possédant les meilleures propriétés de frottements et de résistance à l'abrasion possibles en regard des têtes fémorales utilisées.

Selon une caractéristique de l'invention, l'insert peut être constitué en tout ou partie d'un matériau métallique, céramique ou polymère choisi parmi : un acier inoxydable , un alliage cobalt-chrome, une céramique d'alumine, une céramique de zircone, un mélange alumine-zircone, un polyétheréthercétone , dont au moins la partie constituant l'interface avec l'élément intermédiaire est dotée de cavités ou d'interstices pour constituer l'interface avec l'élément intermédiaire. De manière optionnelle, l'insert peut être fait en un seul matériau (métal ou céramique) ou en plusieurs matériaux associés.

A titre non limitatif, on indique que les matériaux communément utilisés à ce jour sont : acier inoxydable, les alliages cobalt-chrome, la céramique d'alumine, la céramique de zircone, les mélanges alumine zircone ou le polyétheréthercétone. Ce dernier peut être chargé de microfibres de carbone pour améliorer ses caractéristiques mécaniques et tribologiques.

Dans un mode de réalisation particulier, la surface de l'insert est traitée par micro abrasion, pour présenter un relief, formant des cavités ou des interstices, et est imprégnée par le matériau de l'élément intermédiaire. Par exemple, la surface est traitée par sablage par projection de microbilles céramiques. Les modifications de structure microscopiques apportées à la surface de l'insert réalisent une succession d'aspérités en surface pour permettre une interpénétration de la surface de l'insert avec la surface de l'élément intermédiaire par blocage mutuel.

Dans un autre mode particulier de réalisation, l'interface de l'insert avec l'élément intermédiaire adopte une structure poreuse. Par exemple, dans le dispositif selon l'invention, les pores des couches poreuses ont avantageusement un diamètre moyen compris entre 50 µm et 500 µm. Le diamètre est de préférence compris entre 150 µm et 250 µm. Une telle structure poreuse peut être obtenu par exemple par frittage.

Ces procédés peuvent aussi bien être utilisés pour ajouter une couche métallique poreuse sur un insert en polymère ou en céramique. Ils permettent d'ajouter progressivement du métal, chaque ajout apportant quelques microns de matière seulement. Il est alors possible de construire des pièces dont les détails de structure sont de taille à peine supérieure à leur épaisseur. On peut ainsi obtenir une partie métallique à la fois dense et poreuse en surface comprenant différentes couches fonctionnelles, et cependant de faible épaisseur, telle que mise en oeuvre dans le matériau composite selon l'invention. La porosité ainsi créée pourra être soit ordonnée, soit aléatoire, ceci indépendamment du type de frittage utilisé.

L'insert est uni à l'élément intermédiaire par imprégnation du matériau polymère constitutif de l'élément intermédiaire dans la porosité de la couche proximale métallique, de sorte qu'il n'y a pas de limite stricte entre les deux éléments, mais recouvrement sur une zone d'interface, dont l'épaisseur peut atteindre au maximum celle de la structure poreuse.

Les termes "proximal" et "distal" appliqués communément à un élément anatomique correspondent respectivement à la partie la plus rapprochée et à la partie la plus éloignée du tronc. Dans le cas présent, la partie du cotyle la plus proche du bassin est la partie proximale, à l'inverse la partie la plus proche de la tête du fémur est la partie distale.

Les diamètres intérieurs d'insert usuellement utilisés sont compris entre 28 mm et 40 mm généralement 28 mm, 32 mm, 36 mm, 40 mm et sans limite de taille selon l'évolution des usages cliniques. Cela correspond à un diamètre extérieur du cotyle compris entre 44 mm et 68 mm. Dans le cas des cotyles de diamètres commun, par exemple compris entre 44 mm et 54 mm, la couche proximale de l'insert peut être de forme simple et revêtue de métal poreux par exemple, alors que pour les tailles plus importante, dont le diamètre est compris entre 56 mm et 68 mm, l'insert peut être avantageusement pourvu de rainures.

C'est pourquoi, selon un autre mode de réalisation particulier, l'interface de l'insert avec l'élément intermédiaire adopte une structure rainurée. Ces rainures, de taille macroscopique par exemple de quelques dizaines de µm à environ 2 mm, seront occupées par le matériau de l'élément intermédiaire de sorte qu'elles réalisent une interpénétration de la surface de l'insert avec la surface de l'élément intermédiaire par blocage mutuel.

Ainsi, de manière avantageuse selon l'invention, l'interface de l'insert avec l'élément intermédiaire adopte une structure choisie parmi : une structure poreuse, une structure rainurée.

De préférence, l'insert est doté à sa surface en contact avec l'élément intermédiaire, de nervures comportant des évidements. Il est connu de recouvrir à des nervures pour renforcer la rigidité des implants, notamment des implants de grande taille (diamètre supérieur à 44 mm). Ces nervures pouvant servir de renfort de la rigidité de l'insert, peuvent commodément comporter des évidements qui permettent de réaliser une interpénétration entre le matériau composant l'insert et l'élément intermédiaire par comblement desdits évidements par ledit matériau polymère. Ces nervures évidées consolident alors l'accrochage entre l'élément intermédiaire et l'insert et constituent un rempart contre les phénomènes d'arrachement et de rotation de l'insert. De manière alternative on peut également envisager tout autre type de structures en relief propres à offrir un ancrage efficace entre l'insert et le matériau polymère de l'élément intermédiaire.

Préférentiellement, l'insert est réalisé en matériau polymère recouvert de métal poreux à sa surface en contact avec l'élément intermédiaire. Dans ce cas, l'insert, obtenu de manière commune par injection, est recouvert de métal poreux par torche plasma.

Selon des modes préférés de réalisation de l'invention, l'insert est réalisé en polyétheréthercétone (chargé ou non de micro fibres de carbone). La surface de l'insert est traitée par micro abrasion, pour présenter un relief, formant des cavités ou des interstices ou recouverte de titane poreux à sa surface en contact avec l'élément intermédiaire. Le polyétheréthercétone ou PEEK est un matériau bien connu de l'homme du métier pour sa résistance à l'abrasion et son module d'élasticité proche de celui de la structure osseuse.

La cupule est l'élément de l'implant servant à fixer l'implant dans l'os. Elle va aussi coopérer avec l'insert, pour maintenir la tête fémorale prothétique. La forme extérieure générale de la cupule et plus généralement de l'implant, peut concerner tous les cotyles classiques dans les versions à bords plats ou à débord anti-luxation déjà connu de l'homme du métier. Elle est classiquement de forme essentiellement hémisphérique, afin de répartir les contraintes de manière uniforme

Le métal constituant la cupule peut être un métal pur ou un alliage, choisi parmi les métaux de qualité médicale, connus pour leur compatibilité avec les tissus biologiques. De préférence, la cupule est réalisée en tout ou partie d'un matériau métallique choisi parmi : le titane, le tantale, le niobium, un alliage de cobalt et de chrome, un acier inoxydable, un alliage métallique comprenant l'un de ceux-ci, ledit matériau constituant aussi l'interface avec l'élément intermédiaire. Selon une caractéristique particulière, la cupule peut être réalisée intégralement en en titane, en un alliage métallique de titane, tel qu'un alliage ternaire de titane, d'aluminium et de vanadium (communément désigné TA6V), ou encore en alliage cobalt-chrome.

Dans un mode particulier de réalisation, l'interface de la cupule avec l'élément intermédiaire adopte une structure poreuse. Cette structure poreuse est apte à réaliser une interpénétration de la cupule avec l'élément intermédiaire par imprégnation de la porosité par le matériau polymère. En outre, la cupule peut aussi être poreuse sur la face d'accroche à l'os. Dans ce cas, selon l'invention, les deux couches superficielles de la cupule présentent des structures poreuses. Ainsi, comme expliqué plus haut, la cupule comprend, j) deux couches superficielles poreuses et dont l'une, distale, constitue l'interface de la cupule avec l'élément intermédiaire et l'autre, proximale, est destinée à assurer la fixation dans ladite structure osseuse, et jj) une couche médiane ladite couche médiane étant dense de sorte que celle-ci constitue une cloison étanche entre lesdites deux couches superficielles. On a ainsi un élément d'accroche sur le tissu osseux (la cupule) extrêmement fin.

En effet, par les techniques précitées, on peut également ajouter le métal de manière discontinue pour créer un réseau de pores de maille prédéterminée. Les inventeurs ont mis à profit cette finesse d'exécution pour choisir la taille et la densité de la porosité la plus adaptée à la fonction prévue des couches superficielles. Il s'agit en particulier de favoriser la pénétration et l'accroche des cellules osseuses d'une part et du polymère d'autre part, sans fragiliser la structure. Après installation de l'implant, la couche superficielle poreuse sera colonisée par les cellules osseuses, ce qui assurera une fixation efficace et durable de l'élément prothétique. Cette fixation osseuse pourra être avantageusement améliorée par une imprégnation d'hydroxyapatite par dépôt nanométrique en phase aqueuse en surface et dans l'épaisseur en tapissant l'intérieur des porosités. Cette technique biomimétique respecte l'interconnexion des porosités. Cependant, du fait de la cloison formée par la couche médiane, les cellules osseuses pourront coloniser la porosité de la couche superficielle proximale, sans traverser la cupule dans toute son épaisseur. Dans le dispositif selon l'invention, la cupule peut avoir une épaisseur totale allant de 0,5 mm à 2,0 mm, et de préférence de 1,0 mm à 1,5 mm.

A noter qu'il est aussi possible de faire varier la nature du métal apporté au cours de la fabrication. De manière avantageuse, la couche superficielle proximale de la cupule a une épaisseur comprise entre 0,20 mm et 0,60 mm et son épaisseur est comprise entre 0,35 mm et 0,45 mm ; la couche superficielle distale de la cupule a une épaisseur comprise entre 0,20 mm et 0,60 mm, et de préférence entre 0,35 mm et 0,45 mm ; la couche médiane de la cupule a une épaisseur comprise entre 0,10 mm et 0,80 mm et de préférence, elle est dans un intervalle compris entre 0,30 mm et 0,60 mm.

Chaque couche de la cupule est donc d'épaisseur réduite, ce qui permet d'obtenir une structure mince tout en offrant d'excellentes caractéristiques concernant la rigidité et les propriétés adhésives. La mise en oeuvre de cette cupule permet d'accueillir une épaisseur maximum du matériau polymère composant l'élément intermédiaire. Elle permet également de concevoir des implants avec un choix de formes et de dimensions accru, tout en répondant plus aisément aux différentes contraintes de conformation imposées par l'organisation spatiale et la cinématique des articulations.

En outre, la cupule peut avantageusement comporter des aspérités ou des trous sur sa face externe, pour la fixation à l'os par encastrement (press-fit) ou par vissage. Ces aspérités s'étendent avantageusement sur une zone de 1 à 2 centimètres sur la face proximale de la cupule en partant du bord de celle-ci. La bordure de la cupule désigne la partie qui forme le pourtour de l'hémisphère constituant la cupule. De la même manière la bordure de l'insert désigne la partie qui forme le pourtour de l'hémisphère constituant l'insert. Les aspérités permettent dans le cas d'un encastrement en force de l'implant d'offrir une plus grande surface pour la colonisation osseuse et assurent ainsi une meilleure adhésion de l'implant dans l'os. La surface extérieure poreuse peut en plus être avantageusement imprégnée d'hydroxyapatite nanométrique pour dynamiser la fixation osseuse. L'imprégnation se fait par voie aqueuse et non par torche plasma qui a l'inconvénient de boucher les pores.

De préférence, l'élément intermédiaire est en matériau polymère reconnu de qualité implantable choisi parmi un polyéthylène, un polyéthylène de haute masse moléculaire, un silicone ou un polycarbonate uréthane.

On peut utiliser par exemple un polyéthylène de haute masse moléculaire, appelé couramment UHMW PE, avec ou sans apport de vitamine E (pour limiter l'oxydation), et réticulé ou non. Sa masse moléculaire peut être avantageusement comprise entre 2.10⁶ et 6.10⁶. Ce type de polymère est communément utilisé pour la réalisation de prothèses, et l'homme de l'art sait choisir la qualité adéquate dans le catalogue des fournisseurs spécialisés. Le polymère se présente généralement sous forme d'une masse dense homogène, obtenue à partir de poudre qui, lorsqu'elle est compressée à chaud, fond et adopte la forme de son contenant. C'est cette propriété qui est mise à profit pour créer des interfaces interpénétrées avec l'insert et la cupule respectivement.

La cupule et l'insert ayant une forme essentiellement hémisphérique où Dc correspondant au rayon interne de la cupule et Di au rayon externe de l'insert, la distance Dc-Di définit un espace E qui est occupé par l'élément intermédiaire. Comme on vient de le voir, par la liaison indéfectible de ses différentes parties, l'implant selon l'invention interdit les micro-déplacements d'une partie par rapport à l'autre et limite la formation de débris de l'élément intermédiaire en matière polymère. Pour éviter tout risque, il a été prévu que le dispositif assure en plus un confinement total de l'élément intermédiaire. En effet, si la cupule et l'insert constituent des parois infranchissables, leurs bordures peuvent encore représenter une faille à la sortie de débris de l'élément intermédiaire. Un autre problème réside dans le processus de fabrication au cours duquel le polymère en fusion risque de déborder de l'espace qui lui est imparti.

C'est pourquoi, dans un mode de réalisation particulièrement intéressant de l'invention, la cupule et l'insert sont munis de moyens de jonction étanche entre eux, de sorte que l'élément intermédiaire est confiné entre la cupule et l'insert. Cette jonction étanche permet d'isoler totalement l'élément intermédiaire de l'extérieur de l'implant.

Les moyens de jonction peuvent adopter différentes formes. Selon un mode de réalisation particulier de l'invention, la cupule et l'insert adoptent une forme essentiellement hémisphérique de rayon respectif Dc et Di, avec Dc > Di, la distance Dc - Di définissant un espace E occupé par l'élément intermédiaire, l'insert ayant une bordure qui se prolonge par une extension périphérique dans le plan de l'équateur formant une collerette de largeur Dc - Di qui ferme ledit espace E par une jonction étanche. De préférence, le bord externe de ladite collerette de l'insert est engagé par emboîtement en serrage contre la face interne de la bordure de la cupule et forme une jonction étanche.
L'implant qui vient d'être décrit apporte des performances élevées en termes de confort et de longévité, grâce à la faible épaisseur de la cupule permettant l'épaisseur élevée de l'élément intermédiaire, ce qui confère à la prothèse une plus grande élasticité et un meilleur amortissement des chocs. Cet avantage est démultiplié par les caractéristiques de porosité que l'on peut donner à la cupule et l'insert. Il est fabriqué en utilisant les propriétés thermoplastiques du polymère, condition pour que les interfaces interpénétrées puissent se former. Les technologies de thermocompression sont donc tout indiquées. Toutefois de nombreux problèmes techniques se posent pour fabriquer un tel implant. Il est en effet difficile d'obtenir la fusion d'un matériau polymère qui est enserré entre deux parois rigides. Le contrôle de la température est délicat. La pression appliquée est également source de problèmes, les éléments rigides que sont la cupule et l'insert risquant de se déformer de manière irréversible et totalement indésirable durant le processus de thermoformage. On redoute également que le polymère ne déborde de l'espace qui lui est réservé lors de l'assemblage de la cupule et de l'insert. C'est pourquoi un procédé a été mis au point pour résoudre ces problèmes, et permettre la fabrication de l'implant décrit plus haut.

L'objet de l'invention peut être obtenu par un procédé de fabrication d'un implant articulaire destiné à être fixé dans une structure osseuse et à accueillir une tête prothétique, mobile par rapport à ladite structure osseuse, l'implant comprenant i) une cupule en métal assurant la fixation de l'implant dans ladite structure osseuse, ii) un insert assurant la réception de ladite tête prothétique, et iii) un élément intermédiaire en matériau polymère, interposé entre la cupule et l'insert, le procédé comprenant les étapes consistant à :
a) se munir d'un insert dont la surface qui sera en contact avec l'élément intermédiaire présente un relief formant des cavités ou des interstices,
b) assembler par thermocompression un matériau polymère sous forme de poudre contre la face en relief de l'insert, pour obtenir une première pièce dans laquelle le matériau polymère occupe au moins en partie les cavités ou interstices formant le relief de l'insert de sorte que le matériau et l'insert sont unis par une interface interpénétrée,
c) usiner le matériau polymère pour obtenir une deuxième pièce dont le matériau polymère adopte une forme, préfigurant celle de l'élément intermédiaire désiré, propre à se fixer à l'intérieur de la cupule,
d) se munir d'une cupule en métal présentant un relief formant des cavités ou des interstices à la surface qui sera en contact avec l'élément intermédiaire,
e) assembler par thermocompression le matériau polymère de ladite deuxième pièce (la face libre) contre la face portant le relief de ladite cupule, pour obtenir une troisième pièce dans laquelle le matériau polymère occupe au moins en partie les cavités ou interstices formant le relief de la cupule, de sorte que le matériau et la cupule sont unis par une interface interpénétrée, ladite troisième pièce ainsi formée constituant un implant articulaire selon l'invention.

L'assemblage de l'implant est donc conduit en deux opérations successives de thermocompression. Au cours de la première opération, le polymère en fusion est mis en contact avec la surface distale de la cupule et pénètre dans la porosité ou entre les interstices sur une épaisseur pouvant varier en fonction de différents paramètres, essentiellement la température, la pression et la durée de la compression. Après refroidissement, on obtient un matériau composite indissociable. Au cours de la deuxième opération, le polymère en fusion est mis en contact avec la surface proximale de l'insert et pénètre dans la porosité ou entre les interstices.

Préférentiellement, à l'étape b), l'insert et le polymère en poudre sont placés dans un moule de thermocompression comportant une chambre de compression formée d'un socle en partie inférieure, d'une chemise cylindrique latérale comportant un alésage cylindrique dans lequel est monté en coulissement un piston, la poudre étant au contact du relief formant des cavités ou des interstices dudit insert, et le moule est mis sous pression et est porté à une température T₁ supérieure à la température de fusion du polymère jusqu'à la prise en masse de la poudre de polymère et pénétration dans les cavités ou interstices de l'insert, pour constituer ladite première pièce.

Cette première étape de thermocompression destinée à porter à sa température de fusion l'intégralité de la poudre thermoplastique se fait une température voisine de la température de fusion du polymère. La maîtrise des paramètres de thermocompression doit permettre d'obtenir la fusion complète et en masse du polymère pour qu'il pénètre dans les cavités ou interstices de l'insert. Cette étape est suivie du refroidissement du matériau polymère jusqu'à sa solidification.

A l'issue de cette première opération de thermocompression, on dispose d'un bloc de polymère associé à un insert. Le bloc ayant la forme du moule, il convient de l'amener à des forme et dimensions proches de celle de la cupule avec laquelle il va ensuite être associé, avant de constituer l'élément intermédiaire désiré. Ceci peut être fait par différentes techniques, de préférence par tournage à l'aide d'un outil conventionnel.

La pièce est alors prête pour subir la seconde opération de thermocompression. De préférence à l'étape e), la deuxième pièce et la cupule sont placées dans un moule de thermocompression comportant une chambre de compression formée d'un socle en partie inférieure, d'une chemise cylindrique latérale comportant un alésage cylindrique dans lequel est monté en coulissement un piston, le matériau polymère étant en contact du relief formant les cavités ou interstices de ladite cupule, et la chambre de compression est mise sous pression et est portée à une température T₂ voisine de la température de fusion du polymère jusqu'à la formation d'une pâte de polymère et pénétration dans les cavités ou interstices de la cupule. Lorsque la cupule adopte une structure en trois couches, une couche médiane dense étant entourée de couches superficielles poreuses, la couche superficielle distale de la cupule est imprégnée par le polymère, sans qu'il puisse pénétrer plus profondément, puisque la couche dense réalise une barrière à l'expansion du polymère.

Cette seconde opération de thermocompression, destinée à fondre uniquement la couche superficielle du matériau polymère de l'élément intermédiaire pour qu'il pénètre dans les cavités ou interstices de la cupule, se fait à une température inférieure à celle nécessaire à la fusion totale du matériau et pendant un temps plus bref. La maîtrise des paramètres de thermocompression doit permettre d'obtenir un ramollissement du polymère composant l'élément intermédiaire uniquement en surface, suffisant pour la pénétration de la matière dans les cavités ou interstices de la cupule. Pour ce fait, selon un mode de réalisation intéressant, la cupule est placée à l'envers dans le fond du moule, c'est-à-dire sa face concave (portant les cavités ou interstices) en regard du bloc thermocomprimé de la pièce obtenue durant la phase b), cette dernière étant placée au-dessus. C'est sur elle que va s'exercer la pression du piston durant la seconde étape de thermocompression.

De manière avantageuse, à l'étape e), la seconde pièce et la cupule sont placées dans un moule de thermocompression comportant une chambre de compression formée d'un socle en partie inférieure, d'une chemise cylindrique latérale comportant un alésage cylindrique dans lequel est monté en coulissement un piston, puis la cupule est placée sur le socle dans le fond du moule, sa face portant le relief tournée vers le haut, et la deuxième pièce est placée au-dessus, le matériau polymère étant en contact du relief formant les cavités ou interstices de ladite cupule, et la chambre de compression est mise sous pression et est portée à une température T2 voisine de la température de fusion du polymère jusqu'à la formation d'une pâte de polymère, jusqu'à la formation d'une pâte de polymère en surface du matériau polymère et pénétration dans les cavités ou interstices du relief de la cupule.

En d'autres termes, préférentiellement, à l'étape e),
- la cupule est placée sur le socle dans le fond du moule, sa face portant le relief tournée vers le haut, et la deuxième pièce est placée au-dessus, puis
- la chambre de compression est portée à une température inférieure d'environ 10° à la température de fusion dudit matériau polymère, jusqu'à la formation d'une pâte de polymère en surface du matériau polymère et pénétration dans les cavités ou interstices de la cupule. L'homme du métier choisira la pression adaptée à la thermocompression du matériau polymère choisi. Cette étape se fait donc sans ajout de poudre polymère. Elle
est suivie du refroidissement du matériau polymère jusqu'à sa solidification et solidarisation de l'ensemble.

De manière avantageuse, préalablement à l'étape e), on place sur le socle au fond du moule un support rigide et conformé à la cote de la cupule, de façon à soutenir ladite cupule durant l'étape e) de thermocompression. En effet, la partie bombée de la cupule étant tournée vers le fond du moule, il est recommandé, pour éviter la déformation de la pièce du fait de la pression exercée sur elle, de placer au préalable au fond du moule un support rigide conformé à la cote de la cupule, qui va soutenir celle-ci durant la thermocompression.

De préférence, ledit support rigide comprend une coque externe en matériau conducteur thermique dans laquelle est logé un élément chauffant, apte à communiquer la température T2 à la surface du matériau polymère à travers la cupule, de manière homogène, c'est-à-dire que la chaleur fournie par l'élément chauffant est répartie sur toute la surface en contact. Cette disposition permet aussi de localiser la zone de chauffe à la surface afin de ne pas faire fondre tout le polymère, et de ne pas déformer la pièce. On pourra choisir un matériau conducteur thermique parmi ceux connus par l'homme du métier notamment l'aluminium. On combine avantageusement des deux caractéristiques.

Selon une autre caractéristique intéressante c'est lors de l'étape e), que les moyens de jonction étanche dont la cupule et l'insert sont munis, sont assemblés sous l'effet de la pression, de sorte qu'à l'issue de cette étape, l'élément intermédiaire est confiné entre la cupule et l'insert. Pour ce faire, on peut choisir une cupule et un insert adoptant une forme essentiellement hémisphérique de rayon respectif Dc et Di, avec Dc > Di, la distance Dc - Di définissant un espace E qui sera occupé par l'élément intermédiaire, la bordure de l'insert se prolongeant par une extension périphérique dans le plan de l'équateur et formant une collerette de largeur Dc - Di, et lors de l'étape e) lorsque le matériau polymère de ladite deuxième pièce vient en contact contre la face en relief de ladite cupule, de manière concomitante, le bord externe de ladite collerette s'engage par emboîtement en serrage contre la face interne de la bordure de la cupule, et ferme ainsi ledit espace E par une jonction étanche.

De préférence, à l'étape c), l'usinage du matériau polymère de la première pièce est réalisé par une opération de tournage, à l'issue de laquelle on conserve une épaisseur de polymère Dc - Di, augmentée d'1 mm qui fournira la quantité de polymère pénétrant dans le relief de la cupule durant l'étape e). Ainsi, la cupule et insert sont assemblés avec précision, sans lacune ni débordement de matière.

Grâce à ce procédé, l'implant objet de l'invention peut être fabriqué en conditions industrielles. Il peut en outre être validé mécaniquement par différents tests de friction, torsion interne, compression équatoriale et simulateur, selon les normes en vigueur. Il sera utilisé par les patients en toute sécurité. Le polymère étant interposé entre l'insert et le cotyle ou même pouvant être totalement confiné à l'intérieur du dispositif, il n'existe aucun risque d'érosion ni de dispersion de débris dans l'organisme. La liaison mécanique de la cupule, de l'insert et de l'élément intermédiaire est très efficace, voire absolue. Le frottement entre la cupule ou l'insert et le polymère de l'élément intermédiaire est inexistant, et donc l'usure associée disparaît. La raideur de l'ensemble peut être modulée dans un large intervalle. Cette structure particulière de l'implant lui confère des propriétés qui sont similaires à celles de l'articulation naturelle.

La présente invention sera mieux comprise, et des détails en relevant apparaîtront, grâce à la description qui va être faite de variantes de réalisation, en relation avec les figures annexées, dans lesquelles :
- La figure 1 est une vue en coupe d'un cotyle prothétique selon l'invention et d'une tête prothétique venant coopérer avec le cotyle (les échelles sont différentes).
- La figure 2A est une vue en coupe (A1) et en perspective (A2), d'un insert selon l'invention à nervures de renfort avec des évidements.
- La figure 2B est une vue en coupe d'un insert selon l'invention à structure rainurée.
- La figures 3 et 4 représentent schématiquement les étapes d'assemblage d'un insert selon l'invention.

### EXEMPLE 1 : Cotyle de hanche à insert poreux

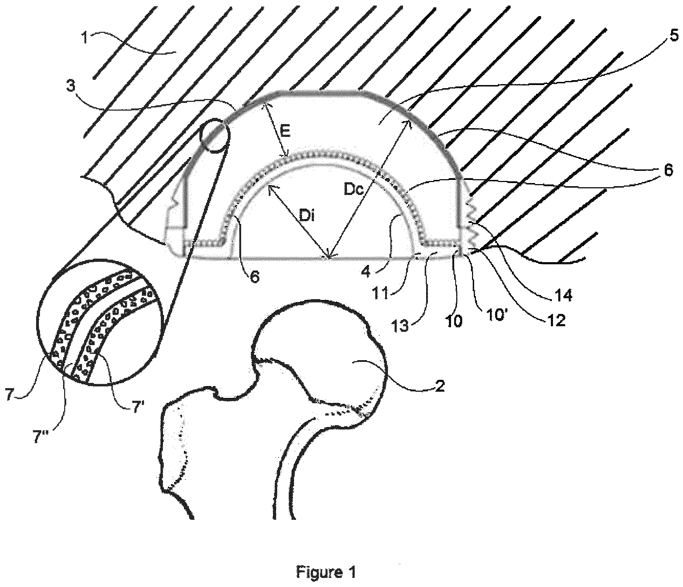

L'implant illustré par la Figure 1 est un cotyle monobloc destiné à être fixé dans la hanche 1 et à accueillir la tête prothétique d'un fémur. Il comprend la cupule 3 assurant la fixation dans l'os, l'insert 4 assurant la réception de la tête 2 et l'élément intermédiaire 5 interposé entre la cupule 3 et l'insert 4. La cupule 3 et l'insert 4 présentent à leur surface de contact avec l'élément intermédiaire 5 un relief 6 formant des cavités. Les surfaces respectives de l'élément intermédiaire 5 épousent le relief 6, réalisant des interfaces interpénétrées entre l'élément intermédiaire 5 et la cupule 3 d'une part, et entre l'élément intermédiaire 5 et l'insert 4 d'autre part.

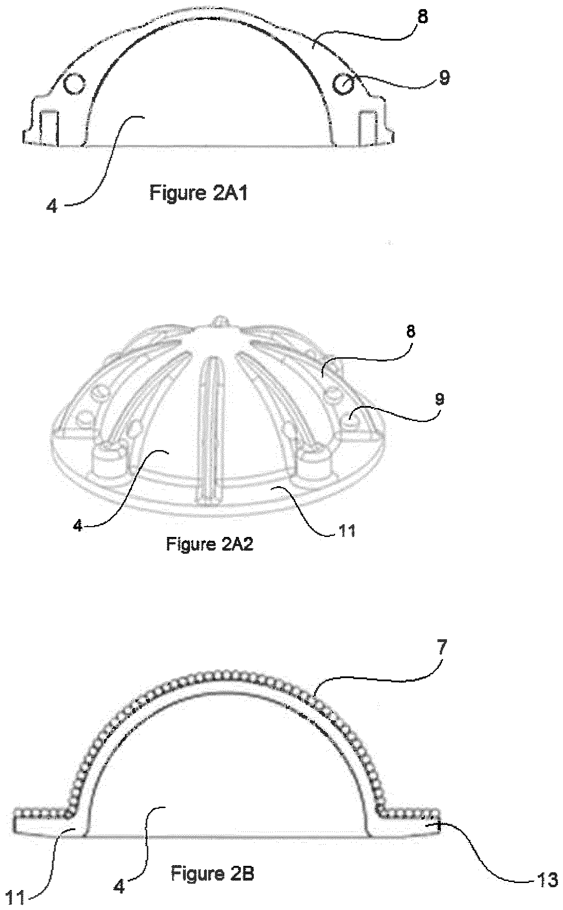

L'insert 4 (figure 2B) est réalisé en polyétheréthercétone (PEEK, qui peut être chargé de particules carbone pour améliorer ses caractéristiques mécaniques et tribologiques), et son interface avec l'élément intermédiaire est recouverte de titane poreux. Ce revêtement de titane poreux est obtenu par projection plasma de poudre de titane. La couche poreuse de l'insert peut avoir une épaisseur de 0,45 mm et des pores de 200 µm. L'autre face, qui sera en contact avec la tête du fémur, est lisse.

La cupule est réalisée en alliage ternaire de titane, d'aluminium et de vanadium. Est utilisé dans le présent exemple un alliage de qualité médicale, dit TA6V, à base de titane de type α + β, d'aluminium (pour 6%) et de vanadium (pour 4%). Elle est ici constituée par trois couches successives réalisées dans le même alliage, à savoir deux couches superficielles poreuses 7' et 7", dont l'une 7", constitue l'interface de la cupule avec l'élément intermédiaire 5, et l'autre 7' est destinée à assurer la fixation dans la hanche 1, et une couche médiane 7'". Cette couche médiane est dense de sorte qu'elle constitue une cloison étanche entre les deux couches superficielles poreuses 7' et 7".

La porosité de la couche 7" s'étend sur toute la surface en contact avec l'élément intermédiaire 5, sauf pour ce qui concerne une bande de la bordure interne 10' qui reste lisse, ce qui permettra de créer une jonction étanche. La porosité de la couche 7' s'étend sur toute la surface en contact avec l'os 1, sauf pour ce qui concerne une bande le long de la bordure 12 qui comporte des aspérités 14, pour favoriser son insertion initiale dans l'os de la hanche.

Dans le cas d'espèce, la cupule 3 a une épaisseur totale de 0,50 mm, avec
- la couche superficielle proximale a une épaisseur de 0,20 mm et des pores de 150 µm ;
- la couche médiane a une épaisseur de 0,30 mm ;
- la couche superficielle distale a une épaisseur de 0,20 mm et des pores de 250 µm. Les couches superficielles de la cupule peuvent être obtenues par un procédé de fabrication rapide tel que le frittage de poudres par faisceau d'électrons, pour obtenir les deux couches poreuses (ou trabéculées) et une couche dense.
Une telle structure poreuse peut être obtenue par une technique connue de l'homme du métier telle que la construction additive directe par laser ("Construction Laser Additive Directe" ou CLAD en anglais) qui repose sur la fusion de poudres métalliques injectées coaxialement à un faisceau laser de puissance pour réaliser des dépôts métalliques par couches successives.
L'élément intermédiaire 5 est en polyéthylène à haut poids moléculaire (UHMW PE), de masse moléculaire comprise par exemple entre 2 et 6 millions. Il peut contenir en outre de la vitamine E à raison de 1 g par kg de polyéthylène et il est hautement réticulé. Il est choisi dans un catalogue de fabricant à la disposition de l'homme du métier.

L'agencement de ces trois éléments constitutifs de l'implant est réalisé comme suit. La cupule 3 et l'insert 4 sont munis de moyens de jonction étanche entre eux, de sorte que l'élément intermédiaire est confiné entre la cupule 3 et l'insert 4. Comme on le voit sur la figure 1, la cupule 3 et l'insert 4 adoptent une forme essentiellement hémisphérique de rayon respectif Dc et Di. La distance Dc - Di définit l'espace E occupé par l'élément intermédiaire 5. On a par exemple Dc = 44 mm et Di = 28 mm, E ayant alors une épaisseur de 16 mm. La bordure 11 de l'insert se prolonge par une extension périphérique dans le plan de l'équateur formant la collerette 13 de largeur Dc - Di qui ferme l'espace E. Le bord externe 10 de la collerette 13 est engagé par emboîtement en serrage contre la face interne 10' de la bordure 12 de la cupule 3 de façon à former une jonction étanche. L'élément intermédiaire 5 est ainsi totalement confiné entre la cupule 3 et l'insert 4.

### EXEMPLE 2 : Cotyle de hanche à insert rainuré

Le mode de réalisation présenté dans cet exemple est un cotyle monobloc destiné à être fixé dans la hanche 1 et à accueillir la tête du fémur 2. Il convient tout particulièrement aux cotyles de grande taille tel un cotyle de hanche de diamètre 68 mm (insert 40 mm). Il comprend une cupule 3, un insert 4 et un élément intermédiaire 5 interposé entre la cupule 3 et l'insert 4, dont les caractéristiques sont voisines de celles exposées à l'exemple 1.

La cupule 3 présente ainsi à sa surface de contact avec l'élément intermédiaire 5 un relief 6 formant des cavités (dans le cas d'espèce il s'agit d'une structure poreuse 7), réalisant une interface interpénétrée entre la cupule 3 et l'élément intermédiaire 5. La cupule peut être identique dans sa structure à celle présentée à l'exemple 1 : elle est réalisée en alliage ternaire de titane, d'aluminium et de vanadium, avec deux couches superficielles poreuses et une couche médiane dense, réalisées dans le même matériau. L'élément intermédiaire 5 est en polyéthylène à haut poids moléculaire, choisi par l'homme de l'art parmi les qualités disponibles dans le commerce pour ce type d'application. L'insert 4, réalisé en polyétheréthercétone (éventuellement chargé de particules carbone), est par contre particulier. Comme illustré aux Figures 2A1 et 2A2, il présente à sa surface de contact avec l'élément intermédiaire 5 un relief 6 formant des rainures comportant des évidements 9. La surface de l'élément intermédiaire 5 épouse ce relief 6, réalisant une interface interpénétrée avec la cupule 3.

L'agencement de ces trois éléments constitutifs de l'implant est réalisé selon le principe exposé à l'exemple 1. La cupule 3 et l'insert 4 adoptent une forme essentiellement hémisphérique de rayon respectif Dc = 34 mm et Di = 20 mm. L'espace E occupé par l'élément intermédiaire a ainsi une épaisseur Dc - Di = 14 mm. La bordure 11 de l'insert se prolonge par une extension périphérique dans le plan de l'équateur formant une collerette 13 de largeur Dc - Di qui ferme ledit espace E par une jonction étanche de sorte que l'élément intermédiaire est confiné entre la cupule 3 et l'insert 4.

### EXEMPLE 3 : Procédé de fabrication

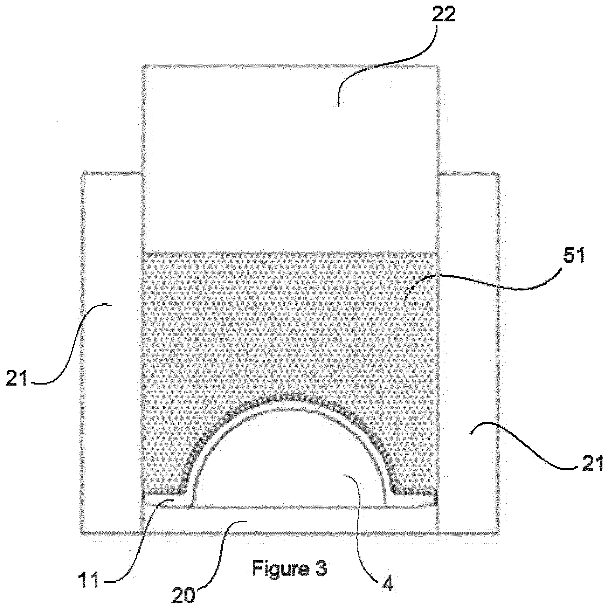

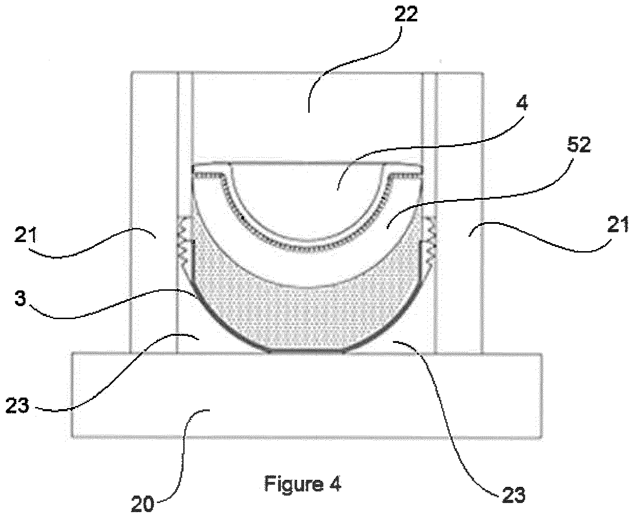

Les implants illustrés aux exemples précédents peuvent être obtenus selon un procédé tel que décrit ci-après, réalisé en deux opérations successives de thermocompression, schématisées aux Figures 3 et 4.

La première opération consiste à assembler par thermocompression un matériau polymère sous forme de poudre contre la face en relief 6 de l'insert 4. On se munit pour ce faire d'un insert 4 tel que décrit précédemment, et du polyéthylène choisi sous forme de poudre 51 ou de compound. L'insert 4 et le polyéthylène en poudre 51 sont placés dans un moule de thermocompression qui comporte une chambre de compression formée du socle 20 en partie inférieure, de la chemise cylindrique latérale 21 comportant un alésage cylindrique dans lequel est monté en coulissement le piston 22. Ce type de moule est communément utilisé dans l'état de la technique.

L'insert 4 est placé dans le moule, sa partie bombée vers le haut, et la poudre 51 est déversée dessus. La poudre est ainsi mise au contact de la porosité ou des interstices de l'insert. Le moule et mis sous pression et est porté à la température T1 comprise entre 200° et 220°C pendant une durée de 5 à 10 minutes, ce qui provoque la fusion du polyéthylène et la formation d'un bloc 52 moulé, une fraction de celui-ci pénétrant dans la porosité ou interstices de l'insert 4. Après refroidissement, on obtient une première pièce dans laquelle le polyéthylène occupe les cavités ou interstices du relief 6 de l'insert 4. On a alors le bloc de polyéthylène pris en masse et l'insert 4 qui sont unis par une interface interpénétrée, de sorte que les deux parties sont indissociables.

Il convient maintenant d'usiner le bloc 52 de polyéthylène, par exemple par tournage, pour lui donner une forme sensiblement complémentaire de celle de la cupule choisie, pour obtenir une seconde pièce. Puis on procède à l'assemblage par thermocompression de la seconde pièce et de la cupule 3. On se munit pour ce faire d'une cupule métallique 3, telle que décrite aux exemples précédentes. On place la seconde pièce et la cupule 3 dans un moule de thermocompression similaire à celui utilisé pour la première opération de thermocompression.

La cupule 3 est placée sur le socle 20 dans le fond du moule, sa face portant la porosité 7" tournée vers le haut, et la deuxième pièce est placée au-dessus, le bloc 52 contre la porosité 7" de la cupule 3. La partie bombée de la cupule est tournée vers le fond du moule. Il est recommandé, pour éviter la déformation de la pièce du fait de la pression exercée sur elle, de placer au préalable au fond du moule un support rigide 23 conformé à la cote de la cupule, qui va soutenir celle-ci durant la thermocompression. Ce support rigide 23 peut comprendre une coque en aluminium dans laquelle est logé un élément chauffant qui va répartir de manière homogène la chaleur dégagée et la communiquer à la surface du polyéthylène, à travers la cupule 3.

Le moule est mis sous pression et est porté à la température T2 comprise entre 160°C et 180°C pendant une durée de 3 à 5 minutes, jusqu'à la formation d'une pâte de polymère à proximité de la zone chaude (sous l'effet notamment de l'élément chauffant en contact de la cupule) et pénétration du polyéthylène dans la porosité de la cupule 3. Le polyéthylène compressé pénètre uniquement dans la couche superficielle 7" de la cupule 3 sur toute son épaisseur jusqu'à la couche médiane 7"' dense qui empêche son expansion plus avant. La zone d'interface est imprégnée par le polyéthylène. Ce faisant, le bloc 52 de polyéthylène ne fond pas et conserve sa conformation générale.

Le polyéthylène constitue ainsi l'élément intermédiaire 5 qui assure une liaison totale entre la cupule et l'insert, ce qui empêche qu'un frottement se produise entre les parties du cotyle prothétique. On obtient une troisième pièce, qui est l'implant selon l'invention, dans laquelle le polyéthylène et la cupule 3 sont unis par une interface interpénétrée. La cupule et l'insert adoptant une forme hémisphérique de rayon respectif Dc et Di, la distance Dc - Di définit un espace E qui est occupé par l'élément intermédiaire 5. Ce dernier occupe un volume important conférant une bonne élasticité à la prothèse, tout en assurant une sécurité totale.

On relève que lors de la seconde opération de thermocompression, une quantité de polyéthylène pénètre dans la porosité de la cupule, de sorte qu'elle ne contribue pas à l'épaisseur de l'élément intermédiaire 5. On anticipe cette consommation de polyéthylène par la porosité lors de l'usinage du bloc 52 : on conserve une épaisseur de polymère Dc - Di augmentée d'1 mm, qui fournira la quantité de polymère pénétrant dans le relief 6 de la cupule 3.

De manière concomitante à ce qui vient d'être décrit, les moyens de jonction étanche dont la cupule 3 et l'insert 4 sont munis sont assemblés sous l'effet de la pression. En effet, la bordure 11 de l'insert se prolonge par une extension périphérique dans le plan de l'équateur formant la collerette 13 de largeur Dc - Di. La bordure 12 de la cupule 3, comporte quant à elle une face interne 10', qui est dépourvue de relief. Durant la compression, le bord externe 10 de la collerette 13 est engagé par emboîtement en serrage contre la face interne 10' de la bordure 12 de la cupule 3, et forme ainsi une jonction étanche. A l'issue de cette étape, l'élément intermédiaire 5 est totalement confiné entre la cupule 3 et l'insert 4. Le polyéthylène est ainsi contenu à l'intérieur de l'implant sans fuite possible vers l'extérieur.

## Revendications

1. Implant articulaire destiné à être fixé dans une structure osseuse (1) et à accueillir une tête prothétique (2) mobile par rapport à ladite structure osseuse, comprenant :
i) une cupule (3) en métal assurant la fixation de l'implant dans ladite structure osseuse,
ii) un insert (4) assurant la réception de ladite tête prothétique, et
iii) un élément intermédiaire (5) en matériau polymère, interposé entre la cupule (3) et l'insert (4),
l'insert (4) présente à sa surface de contact avec l'élément intermédiaire (5) un relief (6) formant des cavités ou des interstices, la surface dudit élément intermédiaire épousant au moins en partie ledit relief,
l'implant étant **caractérisé en ce que**
la cupule (3) comprend,
j) deux couches superficielles (7', 7") présentant un relief (6) formant des cavités ou des interstices, dont l'une, distale, présente une surface de contact avec l'élément intermédiaire (5), la surface dudit élément intermédiaire épousant au moins en partie ledit relief, et l'autre, proximale, est destinée à assurer la fixation de l'implant dans la structure osseuse (1), et
jj) une couche médiane (7"') dense qui constitue une cloison étanche entre lesdites deux couches superficielles,
de manière à réaliser des interfaces interpénétrées entre l'élément intermédiaire (5) et la cupule (3) d'une part, et entre l'élément intermédiaire (5) et l'insert (4) d'autre part.

2. Implant articulaire selon la revendication 1, **caractérisé en ce que** l'insert (4) est constitué en tout ou partie d'un matériau métallique, céramique ou polymère choisi parmi : un acier inoxydable, un alliage cobalt-chrome, une céramique d'alumine, une céramique de zircone, un mélange alumine-zircone, un polyétheréthercétone, dont au moins une partie est dotée d'un relief (6) formant des cavités ou des interstices pour constituer l'interface avec l'élément intermédiaire (5).

3. Implant articulaire selon l'une des revendications 1 ou 2, **caractérisé en ce que** l'interface de l'insert (4) avec l'élément intermédiaire (5) adopte une structure choisie parmi : une structure poreuse, une structure rainurée.

4. Implant articulaire selon l'une des revendications 1 à 3, **caractérisé en ce que** l'insert (4) est doté à sa surface en contact avec l'élément intermédiaire (5), de nervures (8) de renfort comportant des évidements (9).

5. Implant articulaire selon l'une des revendications 1 à 4, **caractérisé en ce que** l'insert (4) est réalisé en matériau polymère recouvert de métal poreux à sa surface en contact avec l'élément intermédiaire (5).

6. Implant articulaire selon l'une des revendications 1 à 5, **caractérisé en ce que** les deux couches superficielles (7', 7") de la cupule (3) présentent des structures poreuses.

7. Implant articulaire selon l'une des revendications 1 à 6, **caractérisé en ce que** l'élément intermédiaire (5) est en matériau polymère choisi parmi un polyéthylène, un polyéthylène de haute masse moléculaire, un silicone, un polycarbonate uréthane.

8. Implant articulaire selon l'une des revendications 1 à 7, **caractérisé en ce que** la cupule (3) et l'insert (4) sont munis de moyens de jonction étanche entre eux, de sorte que l'élément intermédiaire (5) est confiné entre ladite cupule et ledit insert.

9. Implant articulaire selon la revendication précédente, **caractérisé en ce que** la cupule (3) et l'insert (4) adoptent une forme essentiellement hémisphérique de rayon respectif Dc et Di, avec Dc > Di, la distance Dc - Di définissant un espace E occupé par l'élément intermédiaire, l'insert ayant une bordure (11) qui se prolonge par une extension périphérique dans le plan de l'équateur formant une collerette (13) de largeur Dc - Di qui ferme ledit espace E par une jonction étanche.

10. Implant articulaire selon la revendication précédente, **caractérisé en ce que** le bord externe (10) de la collerette (13) de l'insert (4) est engagé par emboîtement en serrage contre la face interne (10') de la bordure (12) de la cupule (3) et forme une jonction étanche.

## Patentansprüche

1. Gelenkimplantat, das dazu ausgelegt ist, in einer Knochenstruktur (1) befestigt zu sein und einen Prothesenkopf (2) aufzunehmen, der bezogen auf die Knochenstruktur beweglich ist, umfassend Folgendes:
i) Eine Pfanne (3) aus Metall, die die Befestigung des Implantats in der Knochenstruktur gewährleistet,
ii) einen Einsatz (4), der die Aufnahme des Prothesenkopfes gewährleistet, und
iii) ein Zwischenelement (5) aus Polymer-Material, das zwischen der Pfanne (3) und dem Einsatz (4) eingefügt ist,
wobei der Einsatz (4) an seiner Kontaktoberfläche mit dem Zwischenelement (5) ein Relief (6) aufweist, das Hohlräume oder Zwischenräume bildet, wobei sich die Oberfläche des Zwischenelements wenigstens teilweise an das Relief anschmiegt,
wobei das Implantat **dadurch gekennzeichnet ist, dass**
die Kuppel (3) Folgendes umfasst:
j) Zwei Oberflächenschichten (7', 7"), die ein Relief (6) aufweisen, das Hohlräume oder Zwischenräume bildet, von denen eines, distal, eine Kontaktoberfläche mit dem Zwischenelement (5) aufweist, wobei die Oberfläche des Zwischenelements sich wenigstens teilweise an das Relief anschmiegt, und wobei das andere, proximal, dazu ausgelegt ist, die Befestigung des Implantats in der Knochenstruktur (1) zu gewährleisten, und
jj) eine dichte mittlere Schicht (7"'), die eine abdichtende Wand zwischen den zwei Oberflächenschichten darstellt, derart, dass sich gegenseitig durchdringende Grenzflächen zwischen dem Zwischenelement (5) und der Pfanne (3) einerseits und zwischen dem Zwischenelement (5) und dem Einsatz (4) andererseits realisiert werden.

2. Gelenkimplantat nach Anspruch 1, **dadurch gekennzeichnet, dass** der Einsatz (4) vollständig oder teilweise hergestellt ist aus einem metallischen, keramischen oder Polymer-Material, das ausgewählt ist aus Folgendem:
Einem rostfreien Stahl, einer Kobalt-Chrom-Legierung, einer Aluminiumoxidkeramik, einer Zirkonkeramik, einer Aluminiumoxid-Zirkon-Mischung, einem Polyetheretherketon, wovon wenigstens ein Teil mit einem Relief (6) versehen ist, das Hohlräume oder Zwischenräume bildet, um die Grenzfläche mit dem Zwischenelement (5) zu bilden.

3. Gelenkimplantat nach einem der Ansprüche 1 oder 2, **dadurch gekennzeichnet, dass** die Grenzfläche des Einsatzes (4) mit dem Zwischenelement (5) eine Struktur annimmt, die aus Folgendem ausgewählt ist: Eine poröse Struktur, eine Rillenstruktur.

4. Gelenkimplantat nach einem der Ansprüche 1 bis 3, **dadurch gekennzeichnet, dass** der Einsatz (4) an seiner Oberfläche in Kontakt mit dem Zwischenelement (5) mit Verstärkungsrippen (8) versehen ist, die Aussparungen (9) umfassen.

5. Gelenkimplantat nach einem der Ansprüche 1 bis 4, **dadurch gekennzeichnet, dass** der Einsatz (4) aus Polymer-Material hergestellt ist, das an seiner Oberfläche in Kontakt mit dem Zwischenelement (5) mit porösem Metall bedeckt ist.

6. Gelenkimplantat nach einem der Ansprüche 1 bis 5, **dadurch gekennzeichnet, dass** die zwei Oberflächenschichten (7', 7") der Pfanne (3) poröse Strukturen aufweisen.

7. Gelenkimplantat nach einem der Ansprüche 1 bis 6, **dadurch gekennzeichnet, dass** das Zwischenelement (5) aus einem Polymer-Material ist, ausgewählt aus einem Polyethylen, einem Polyethylen mit hoher molekularer Masse, einem Silikon, einem Urethan-Polycarbonat.

8. Gelenkimplantat nach einem der Ansprüche 1 bis 7, **dadurch gekennzeichnet, dass** die Pfanne (3) und der Einsatz (4) mit gegenseitig abdichtenden Verbindungsmitteln ausgestattet sind, derart, dass das Zwischenelement (5) zwischen der Pfanne (3) und dem Einsatz (4) begrenzt ist.

9. Gelenkimplantat nach dem vorhergehenden Anspruch, **dadurch gekennzeichnet, dass** die Pfanne (3) und der Einsatz (4) eine im Wesentlichen halbkugelförmige Gestalt mit einem Radius Dc beziehungsweise Di annehmen, wobei Dc > Di ist, wobei der Abstand Dc - Di einen Raum E definiert, der durch das Zwischenelement besetzt ist, wobei der Einsatz eine Umrandung (11) hat, die durch eine Umfangsverlängerung in der Äquatorebene fortgesetzt ist, die einen Kragen (13) mit einer Breite Dc - Di bildet, der den Raum E durch eine abdichtende Verbindung schließt.

10. Gelenkimplantat nach dem vorhergehenden Anspruch, **dadurch gekennzeichnet, dass** der äußere Rand (10) des Kragens (13) des Einsatzes (4) mittels einer Spannverbindung an der Innenfläche (10') der Umrandung (12) der Pfanne (3) angreift und eine abdichtende Verbindung bildet.

## Claims

1. Articular implant intended to be fixed in a bone structure (1) and to accommodate a prosthetic head (2) movable with respect to said bone structure, comprising:
i) a metal cup (3) for fixing the implant in said bone structure,
ii) an insert (4) for receiving said prosthetic head, and
iii) an intermediate element (5) made of polymeric material, interposed between the cup (3) and the insert (4),
the insert (4) has at its contact surface with the intermediate element (5) a relief (6) forming cavities or interstices, the surface of said intermediate element fitting at least in part with said relief,
the implant being **characterized in that**
the cup (3) comprises:
j) two superficial layers (7', 7") having a relief (6) forming cavities or interstices, one of which, distal, has a contact surface with the intermediate element (5), the surface of said intermediate element fitting at least in part with said relief, and the other, proximal, is intended to ensure the fixation of the implant in the bone structure (1), and
jj) a dense median layer (7"') which forms a sealed partition between said two surface layers,
so as to provide interpenetrated interfaces between the intermediate element (5) and the cup (3) on the one hand, and between the intermediate element (5) and the insert (4) on the other hand.

2. Articular implant according to claim 1, **characterized in that** the insert (4) is made entirely or partially of a metallic, ceramic or polymer material chosen from: a stainless steel, a cobalt-chromium alloy, an alumina ceramic, a zirconia ceramic, an alumina-zirconia mixture, a polyetheretheretherketone, at least a part of which is provided with a relief (6) forming cavities or interstices to form the interface with the intermediate element (5).

3. Articular implant according to one of claims 1 or 2, **characterized in that** the interface of the insert (4) with the intermediate element (5) adopts a structure chosen among: a porous structure, a grooved structure.

4. Articular implant according to one of claims 1 to 3, **characterized in that** the insert (4) is provided on its surface in contact with the intermediate element (5) with reinforcing ribs (8) having recesses (9).

5. Articular implant according to one of claims 1 to 4, **characterized in that** the insert (4) is made of polymeric material covered with porous metal on its surface in contact with the intermediate element (5).

6. Articular implant according to one of claims 1 to 5, **characterized in that** the two surface layers (7', 7") of the cup (3) have porous structures.

7. Articular implant according to one of claims 1 to 6, **characterized in that** the intermediate element (5) is made of a polymeric material chosen from polyethylene, high molecular weight polyethylene, silicone, polycarbonate urethane.

8. Articular implant according to one of claims 1 to 7, **characterized in that** the cup (3) and the insert (4) are provided with sealing means between them, so that the intermediate element (5) is confined between said cup and said insert.

9. Articular implant according to the preceding claim, **characterized in that** the cup (3) and the insert (4) adopt an essentially hemispherical shape of respective radius Dc and Di, with Dc > Di, the distance Dc - Di defining a space E occupied by the intermediate element, the insert having an edge (11) which extends by a peripheral extension in the plane of the equator, forming a flange (13) of width Dc - Di which closes said space E by a sealed junction.

10. Articular implant according to the preceding claim, **characterized in that** the outer edge (10) of the flange (13) of the insert (4) is engaged in a tight fitting manner against the inner face (10') of the edge (12) of the cup (3) and forms a sealed junction.
